# EUROPÄISCHE PATENTSCHRIFT

(11) **EP 3 823 707 B1**
(45) Veröffentlichungstag und Bekanntmachung des Hinweises auf die Patenterteilung: **06.09.2023**
(21) Anmeldenummer: 19737665.0
(22) Anmeldetag: 17.06.2019
(51) Int. Cl.: A61M 15/00

(54) **HANDGERÄT ZUR PORTIONIERTEN AUSGABE SPRÜHFÄHIGER SUBSTANZEN**
HAND-HELD DEVICE FOR DISPENSING SPRAYABLE SUBSTANCES IN A PORTIONED MANNER
APPAREIL À MAIN DE DISTRIBUTION EN PORTIONS DE SUBSTANCES PULVÉRISABLES

(30) Priorität: 16.07.2018 DE 102018117106
(43) Veröffentlichungstag der Anmeldung: 26.05.2021
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2019/065888
(87) Internationale Veröffentlichungsnummer: WO 2020/015943

(56) Entgegenhaltungen:
- EP-A1- 0 490 797
- EP-A1- 2 300 084
- EP-A2- 0 414 536
- WO-A1-2004/028608
- WO-A2-2008/142015
- US-A1- 2008 178 872

## Beschreibung

### Gebiet der Technik

Die Offenbarung betrifft ein Handgerät zur portionierten Ausgabe sprühfähiger Substanzen, insbesondere Inhaliermedikamente, mit einem Gehäuse und einer durch eine Druckbeaufschlagung relativ zu dem Gehäuse in eine Ausgabestellung verschiebbaren Kartusche, wobei das Gehäuse ein Mundstück aufweist, wobei weiter die Kartusche zunächst in eine Ausgabebereitschaftsstellung verschiebbar ist und bei der in Ausgabebereitschaftsstellung befindlichen Kartusche durch Ansaugen ein Ausgabesprühvorgang auslösbar ist, wobei weiter die Verschiebung der Kartusche in die Ausgabebereitschaftsstellung durch Spannen einer an dem Gehäuse abgestützten und auf die Kartusche wirkenden Feder durchführbar ist, wobei die auf die Kartusche einwirkende Feder in die Lage versetzt wird, einen Sprühvorgang durchzuführen, hieran aber durch eine Gegenkraft gehindert ist, welche Gegenkraft durch den Benutzer zufolge einer Unterdruckbeaufschlagung durch das Mundstück des Handgerätes außer Wirkung bringbar ist, wobei durch den Unterdruck eine Ventilklappe bewegt wird, die eine mechanische Sperre der Verschiebung der Kartusche aus der Ausgabebereitschaftsstellung in die Ausgabestellung aufhebt.

### Stand der Technik

Handgeräte zur portionierten Ausgabe sprühfähiger Substanzen sind bereits in verschiedenen Ausgestaltungen bekannt. Es wird beispielsweise auf die WO 2009/037085 A1 verwiesen. Derartige Handgeräte finden insbesondere Anwendung in der medizinischen Aerosol-Therapie zur Behandlung von Erkrankungen der Atemwege. Die in dem Gehäuse gehalterte, unter Druck stehende Kartusche beinhaltet das zu inhalierende Medikament, wobei zur Freigabe beziehungsweise zum Ausstoß des Inhaliermedikamentes eine axiale Bewegung der Kartusche relativ zu dem Gehäuse erforderlich ist.

In diesem Zusammenhang sind weiter Ausführungen bekannt, bei welchen, wie auch in dem vorgenannten Stand der Technik, die Kartusche von Hand unmittelbar, so insbesondere im Bodenbereich der Kartusche, zur Bewegung relativ zu dem Gehäuse beaufschlagt wird. Diese manuelle Auslösung erweist sich bei manchen Patienten als problematisch. Zudem ist zur Erzielung einer ordnungsgemäßen Inhalation ein Niederdrücken der Kartusche in dem Gehäuse zur Auslösung des Sprühvorganges zeitgleich mit einem Ansaugen über das Mundstück des Handgerätes vorzunehmen.

Um dieser Problematik zu begegnen, sind weiter Lösungen bekannt, bei welchen die Auslösung des Ausgabesprühvorganges zufolge einer Unterdruckbeaufschlagung durch Ansaugen über das Mundstück des Handgerätes erreicht wird. Die Kartusche ist hierzu zuvor in eine Ausgabebereitschaftsstellung verbracht, in welcher die Kartusche unter Federbeaufschlagung in Richtung auf die Ausgabesprühstellung vorgespannt ist. Eine solche Ausgestaltung ist beispielsweise aus der US 5,447,150 A bekannt. Dabei ist die Kartusche in dem Gehäuse zunächst und im Wesentlichen in einer abgedichteten Kammer geführt, so dass zufolge der zunächst vorgenommenen Verlagerung der Kartusche in die Ausgabebereitschaftsstellung eine Komprimierung der Luft in dem Raum in Verlagerungsrichtung vor der Kartusche erreicht wird. Der ansteigende Luftdruck erzeugt eine Widerstandslast, die die Betätigung des in der Kartusche abgefederten Ventils zur Auslösung des Sprühvorganges hindert. Mit Einsetzen einer Unterdruckbeaufschlagung durch das Mundstück zufolge Ansaugen durch den Benutzer wird eine weiter vorgesehene Ventilklappe bewegt, über welche eine Ventilöffnung freigegeben wird, wobei die in dem vorbeschriebenen Raum komprimierte Luft freigesetzt wird. Durch die weiterhin auf die Kartusche einwirkende Feder wird die Kartusche in diesem Moment weiter in die Ausgabesprühstellung bewegt, mit damit einhergehendem Öffnen des Sprühventils und Ausstoßen der Substanz in den Saugstrom.

Aus der EP 414 536 A2 ist ein Handgerät zur portionierten Ausgabe sprühfähiger Substanzen bekannt bei dem unmittelbar unterhalb einer unteren Stirnfläche eines Substanzbehälters ein Wippenteil angeordnet ist, dass ein weiteres, mit der Ventilklappe beim Niederdrücken zusammenwirkendes Teil bei einer Rückbewegung des Substanzbehälters wieder in eine Ursprungsstellung zurück bewegen kann. Darüber hinaus ist ein in eine Öffnung des Gehäuses eingesetztes Schalterteil vorgesehen, mit dem von Hand eine Aufhebung der Niederdrücksperre, also ohne dass ein Ansaugen erforderlich ist, vorgenommen werden kann. Die Öffnung ist in beiden möglichen Stellungen des Schalters durch den Schalter besetzt.

Aus der EP 490 797 B1 ist ein Handgerät zur portionierten Ausgabe sprühfähiger Substanzen bekannt, bei welchem die Kartusche selbst zur Ausgabe nicht verschoben wird, sondern ein unterhalb der Kartusche angeordneter Schlitten bei Saugbetätigung durch einen Nutzer das Ventil der Kartusche betätigt.

### Zusammenfassung der Erfindung

Ausgehend von einem Stand der Technik gemäß der EP 414 536 A2 beschäftigt sich die Erfindung mit der Aufgabenstellung, ein Handgerät der in Rede stehenden Art vorteilhaft, insbesondere möglichst funktionssicher, auszubilden.

Diese Aufgabe ist beim Gegenstand des Anspruches 1 gelöst, wobei darauf abgestellt ist, dass die Ventilklappe in der Bewegungsrichtung der Kartusche aus der Ruhestellung in die Sprühstellung unterhalb eines ersten luftdurchlässigen Gehäusebodens angeordnet ist und dass der Gehäuseboden einen die Ventilklappe aufnehmenden Raum zu dem Mundstück trennt..

Zufolge der vorgeschlagenen Ausgestaltung wird die Kartusche in ihrer Ausgabebereitschaftsstellung durch rein mechanische Mittel gehalten und so zunächst an der Weiterverlagerung in die Ausgabesprühstellung gehindert. Hieraus ergibt sich eine insbesondere funktionssichere Ausgestaltung. Es bedarf keiner, wie aus dem Stand der Technik bekannt, aufwendigen Abdichtung der Kartuschenumgebung, was auch zu einer vorteilhaften Herstellbarkeit beitragen kann. Bei üblicher, gegebenenfalls häufiger Anwendung des Handgerätes können sich in einem abzudichtenden Bereich Undichtigkeiten einstellen, welche die Funktionssicherheit des Handgerätes beeinträchtigen und darüber hinaus gegebenenfalls das Inhalationsergebnis beeinflussen können.

Die mechanische Sperre kann durch eine mittelbare oder auch unmittelbare mechanische Abstützung der Kartusche an der Ventilklappe bewirkt sein. So kann weiter die mechanische Sperre ein Element darstellen, welches sich zur Hinderung der Weiterverlagerung der Kartusche über die Ausgabebereitschaftsstellung hinaus in die Sprühstellung in den Verlagerungsweg der Kartusche relativ zu dem Gehäuse stellt. Durch die Unterdruckbeaufschlagung über das Mundstück des Handgerätes wird die Ventilklappe derart bewegt, beispielsweise schwenkverlagert, dass die hiermit gekoppelte mechanische Sperre den Verlagerungsweg der Kartusche hin zu der Sprühstellung freigibt.

Die Bewegungsrichtung der Kartusche relativ zu dem die Kartusche aufnehmenden Gehäuse ist bevorzugt linear, weiter bevorzugt gleichgerichtet mit einer Bewegungsrichtung des in einem Inneren der Kartusche vorgesehenen Kartuschenventils zur Sprühausgabe der in der Kartusche befindlichen Substanz. Darüber hinaus ist die Ventilklappe bei üblicher Handhabung des Handgerätes im Zuge eines Inhalationsvorganges, bei welcher Handhabung die Kartusche im Wesentlichen zumindest entlang einer Vertikalachse orientiert ausgerichtet ist, in einem dem Mundstück des Gehäuses im Wesentlichen zugeordneten Bodenbereich des Gehäuses angeordnet.

In der Bewegungsrichtung der Kartusche aus der Ruhestellung in die Sprühstellung ist die Ventilklappe erfindungsgemäß unterhalb eines ersten luftdurchlässigen Gehäusebodens angeordnet. Dieser erste luftdurchlässige Gehäuseboden trennt einen die Ventilklappe aufnehmenden Raum zu dem Mundstück und/ oder dem sich an dem Mundstück anschließenden Luftkanal. Die Luftdurchlässigkeit des ersten Gehäusebodens ist zumindest so weit gegeben, dass eine übliche Luftmenge im Zuge des Ansaugens über das Mundstück durch den ersten Gehäuseboden strömen kann.

Eine gezielte Auslösung der Kartusche, d.h. eine gezielte Verlagerung der Kartusche aus der Ausgabebereitschaftsstellung heraus in die Ausgabestellung kann beispielsweise zu Test- und/oder Diagnosezwecken auch erreicht werden, ohne dass über das Mundstück durch den Benutzer eine Unterdruckbeaufschlagung vorgenommen wird. Der bei der Unterdruckbeaufschlagung das Mundstück umfassende Mund lässt eine Prüfung und gegebenenfalls ein Fotografieren beispielsweise eines Sprühbildes nicht oder nicht in ausreichendem Maß zu. Zu diesem Zweck kann eine alternative nicht erfindungsgemäße Auslösung vornehmbar sein.

So kann vorgesehen sein, dass das Gehäuse eine zusätzlich zu dem Mundstück ausgebildete Öffnung aufweist, durch welche ein Auslöseteil in das Innere des Gehäuses zum Außerwirkungbringen der Gegenkraft einführbar ist, dass die Öffnung eine Bewegung des Auslöseteils zur Einwirkung auf einen im Inneren des Gehäuses angeordneten Auslöseschlitten ermöglicht und dass durch den Auslöseschlitten eine Ventilklappe bewegbar ist.

Es kann hierzu eine gesonderte Öffnung in dem Gehäuse vorgesehen sein. Alternativ kann eine ohnehin vorgesehene, insbesondere zur Durchführung des Inhalationsvorganges vorgesehene Öffnung und/ oder eine zur Verlagerung der Kartusche aus der Ausgabebereitschaftsstellung in die Ausgabestellung über eine Unterdruckbeaufschlagung notwendige Öffnung genutzt werden.

Durch die Öffnung kann ein Auslöseteil derart in das Innere des Gehäuses greifen, dass nach Verlagerung der Kartusche in die Ausgabebereitschaftsstellung mittels des Auslöseteils die die Kartusche in diese Ausgabebereitschaftsstellung haltende Gegenkraft überwunden werden kann, so dass ein Verschieben der Kartusche in die Ausgabestellung und somit ein Ausgabe-Sprühstoß erreicht werden kann.

Zufolge dieser Ausgestaltung ist ein Sprühstoß abgebbar, ohne dass über das Mundstück eine Unterdruckbeaufschlagung vorgenommen wird. Der bei der Auslösung über das Auslöseteil über das Mundstück ausgestoßene Sprühstoß kann auf diese Weise analysiert, vermessen und/ oder auch fotografiert werden.

In weiterer Ausgestaltung kann die Öffnung, durch welche das Auslöseteil einführbar ist, in der Nichtnutzungsstellung des Handgerätes verschlossen sein. So kann die Öffnung in weiter bevorzugter Ausgestaltung erst im Zuge der Verlagerung der Kartusche aus einer Grundstellung hin in die Ausgabebereitschaftsstellung zur Nutzung freigelegt werden.

Zur manuellen Aufhebung der Gegenkraft ist ein Werkzeug (Auslöseteil) notwendig, das gezielt und willensbetont durch die Öffnung eingeführt werden muss. Hierdurch ist eine ungewollte Auslösung verhindert. Auch sind bevorzugt an dem Gehäuse keine Merkmale, beispielsweise in Form einer gesonderten Taste oder dergleichen, vorgesehen, die eine manuelle Auslösung durch den Benutzer herbeiführen könnten.
Das Gehäuse kann eine Sprühdüse aufweisen, aus der in der Sprühstellung der Kartusche die Substanz austritt. Die Sprühdüse des Gehäuses ist in der Nutzungs- beziehungsweise Bereitschaftsstellung des Handgerätes mit einem Ausgabekanal der Kartusche strömungsmäßig verbunden. Über die Sprühdüse wird beim Ausgabesprühvorgang die in der Kartusche bevorratete Substanz portioniert in den zu dem Mundstück führenden Luftkanal oder unmittelbar in den Bereich des Mundstückes gerichtet ausgegeben.

Erfindungsgemäß ist der erste Gehäuseboden luftdurchlässig unterhalb der Sprühdose ausgebildet, und dies im Wesentlichen mit Bezug auf die Bewegungsrichtung der Kartusche aus der Ruhestellung in die Sprühstellung.

Auch kann der erste Gehäuseboden als Teil einer unteren Wandung des Luftkanals gebildet sein, so beispielsweise einen über das Mundstück hinaus in Richtung auf das Gehäuseinnere betreffenden Teil der unteren Kanalwandung.

Der erste Gehäuseboden weist zumindest einen Luftdurchlass auf, gegebenenfalls aber auch eine Mehrzahl von Luftdurchlässen, wobei gemäß einer bevorzugten Ausgestaltung einer oder jeder der Luftdurchlässe im ersten Gehäuseboden kleiner ausgebildet ist als die Ventilklappe. Entsprechend ergeben sich Öffnungsflächen im Bereich der Luftdurchlässe, die in ihrer Erstreckung kleiner gewählt sind als die diesbezüglichen Erstreckungsmaße der Ventilklappe. Somit ist sichergestellt, dass die Ventilklappe nicht in den Bereich des Luftkanals und hierüber in den Bereich des Mundstückes gelangen kann. Entsprechend ist sichergestellt, dass eine gegebenenfalls bei Gebrauch des Handgerätes sich beispielsweise durch Beschädigung aus der Halterung lösende Ventilklappe nicht in den Luftkanal und das Mundstück gelangt und hierüber eingeatmet werden kann. Vielmehr ist die Ventilklappe in dem sich unterhalb des ersten Gehäusebodens gegebenenfalls sich einstellenden Teilraum gefangen.

Die mechanische Sperre der Bewegung der Kartusche, die über die Ventilklappe aufhebbar ist, kann gemäß einer Ausgestaltung durch einen Anschlag gegeben sein. Dieser Anschlag wirkt mittelbar oder unmittelbar zusammen mit einem Abschnitt der Kartusche, insbesondere des Kartuschengehäuses.

Der Anschlag kann zur Freigabe der Bewegung der Kartusche in Richtung auf die Sprühstellung schiebeverlagerbar sein, insbesondere linear verlagerbar. Darüber hinaus kann der Anschlag mit der Ventilklappe verschwenkbar sein, zur Freigabe der Kartuschenbewegung. Die Schwenkachse des Anschlags kann hierbei zugleich die Schwenkachse der Ventilklappe darstellen. Dabei kann die geometrische Schwenkachse der Ventilklappe und/oder des Anschlags quergerichtet sein zu der Bewegungsrichtung der Kartusche.

In weiterer Ausgestaltung kann die Kartusche ein Anschlagteil beaufschlagen. Über dieses Anschlagteil kann die Kartusche in der Ausgabebereitschaftsstellung mittelbar an dem Anschlag der Ventilklappe abgestützt sein. Das Anschlagteil kann ein gegenüber der Kartusche gesondertes, darüber hinaus der Kartusche entsprechend zuordbares Teil sein.

Die Ventilklappe selbst kann den Anschlag aufweisen, so beispielsweise zufolge einer einstückigen, gegebenenfalls darüber hinaus auch materialeinheitlichen Ausgestaltung von Ventilklappe und Anschlag. So kann die Ventilklappe mit dem Anschlag beispielsweise ein Kunststoff-Spritzteil sein. Das Anschlagteil der Kartusche ist entsprechend ausgebildet zur Zusammenwirkung mit der Ventilklappe beziehungsweise mit dem Anschlag der Ventilklappe.

Das kartuschenseitige Anschlagteil kann zur Zusammenwirkung mit dem ventilklappenseitigen Anschlag zumindest einen den Luftkanal der Vorrichtung durchsetzenden Anschlagfortsatz aufweisen. Ein solcher Anschlagfortsatz kann beispielsweise stiftartig gebildet sein. In weiterer Ausgestaltung können mehrere, so insbesondere zwei derartiger Anschlagfortsätze vorgesehen sein, die beidseitig der gehäuseseitigen Sprühdüse verlaufend angeordnet sein können. Im Falle von beispielsweise zwei Anschlagfortsätzen können diese mit einer entsprechenden Anzahl an Anschlägen, die bewegungsgekoppelt sind, zusammenwirken. In einer bevorzugten Ausgestaltung wirken beide Anschlagfortsätze mit nur einem klappenseitigen Anschlag zusammen.

Der Anschlag kann bewegungsfest mit der Ventilklappe verbunden sein. Alternativ ist zwar bezüglich der Verschwenkbarkeit ein mit der Ventilklappe bewegungsgekoppelter Anschlag vorgesehen, der jedoch relativ zu der Ventilklappe beziehungsweise zu Halterungsbereichen für den Anschlag um eine Längsachse des Anschlags rotierbar vorgesehen ist. So kann der Anschlag beispielsweise stab- oder rollenartig ausgeformt sein, mit einer Drehachse parallelverlaufend zu der Verschwenkachse der Ventilklappe und des Anschlags. Dies führt zu einer weiter verbesserten Funktionalität, insbesondere im Zuge der Ventilklappenverschwenkung beim Ansaugen über das Mundstück, so dass im Zuge dieser Verschwenkung der so gestaltete Anschlag verbessert aus der Abstützstellung für das Anschlagteil der Kartusche ausschwenken kann.

Das Anschlagteil kann gemäß einer weiteren Ausgestaltung beispielsweise Teil eines durch die Kartusche bei der Bewegung in die Sprühstellung bewegten Zählwerks sein. Ein solches Zählwerk ist aus der eingangs zitierten Literatur WO 2009/037085 A1 bekannt. Das Zählwerk kann das Ausgabeventilrohr der Kartusche umgebend im Wesentlichen im Bereich zwischen Kartusche und Luftkanal angeordnet sein, wobei sich das Zählwerk mit einem Abschnitt auf einem Gehäuseabschnitt abstützt, zur Relativverlagerung zwischen Komponenten des Zählwerks im Zuge der Bewegung der Kartusche aus der Ruhestellung in die Sprühstellung. Hierdurch wird der Inhaliervorgang erfasst. Der Benutzer kann über ein Sichtfenster oder dergleichen beispielsweise die Anzahl der mindestens noch vorhandenen Sprühstöße ablesen.

Ein Teil des Zählwerks kann sich gemäß einer Ausgestaltung über den gesamt möglichen Verlagerungsweg der Kartusche in dem Gehäuse mitverlagern. Dieser Teilbereich des Zählwerks ist entsprechend mit der Kartusche im Wesentlichen bewegungsgekoppelt und trägt gemäß einer möglichen Ausgestaltung das vorerwähnte Anschlagteil. Das Anschlagteil stellt entsprechend über das Zählwerk eine auf den Anschlag wirkende Verlängerung der Kartusche dar.

In weiterer Ausgestaltung kann das Gehäuse zur Spannung der auf die Kartusche wirkenden Feder ein relativ zu der Kartusche beaufschlagbares Abstützteil für die Feder aufweisen. Das Abstützteil kann in einer weiteren Ausgestaltung Teil des Gehäuses sein, das gegenüber einem feststehenden Teil des Gehäuses in Bewegungsrichtung der Kartusche schiebeverlagerbar angeordnet sein kann. Es kann weiter diesbezüglich eine teleskopartige Anordnung des Abstützteiles und des feststehenden Gehäuseteiles gegeben sein.

In weiterer Ausgestaltung kann das Abstützteil topfartig die Kartusche umgeben, wobei der diesbezügliche Topfboden den bei üblicher Nutzung des Handgerätes nach oben weisenden Kartuschenboden überfangen kann. Der Topfboden des Abstützteils kann hierbei das Widerlager für die auf die Kartusche, insbesondere auf den Kartuschenboden einwirkende Feder anbieten.

In vorteilhafter Weise kann das Handgerät eine Mundstück-Verschlusskappe aufweisen. Diese kann, wie auch bevorzugt, unverlierbar an dem Gehäuse angeordnet sein. Dies bietet die weitere Möglichkeit, die Mundstück-Verschlusskappe mit dem über die Feder auf die Kartusche einwirkenden Abstützteil bewegungszukoppeln. Entsprechend bewirkt eine Verlagerung der Mundstück-Verschlusskappe aus der das Mundstück überdeckenden und schützenden Stellung heraus in die das Mundstück freigebende Stellung und zurück eine bewegungsgekoppelte Linearverlagerung des Abstützteils. So kann sich bei einem Verlagern der Mundstück-Verschlusskappe in eine das Mundstück freigebende Stellung eine über die Feder die Kartusche aus der Ruhestellung in die Ausgabebereitschaftsstellung verbringende Linearverlagerung des Abstützteiles ergeben. Mit der Rückführung der Verschlusskappe in die das Mundstück überdeckende Stellung wird eine entsprechende Rückverlagerung des Abstützteils in eine Grundposition erreicht, unter entsprechender Reduzierung der auf die Kartusche wirkenden Federkraft. In einer möglichen Ausgestaltung ist die auf die Kartusche wirkende Federkraft in einer solchen Grundposition zumindest annähernd gleich Null.

Die Kartusche und das mit dieser im Wesentlichen bewegungsgekoppelte Zählwerk werden insbesondere ausgehend von der Sprühstellung, gegebenenfalls aber auch ausgehend von der Ausgabebereitschaftsstellung, über die kartuschenseitige Ventilfeder in die Ruheposition rückverlagert. So kann weiter gemäß einer möglichen Ausgestaltung diese kartuschenseitige Ventilfeder bereits in der Ausgabebereitschaftsstellung in eine Vorspannung verbracht sein.

Die Bewegungskopplung zwischen Mundstück-Verschlusskappe und Abstützteil kann durch eine Exzenterbeaufschlagung eines Kappenfortsatzes gegeben sein. Über den Exzenter wird eine bevorzugte Schwenkbewegung der Verschlusskappe in eine translatorische Bewegung des Abstützteiles übersetzt. Über die Exzenterbeaufschlagung kann auch gemäß einer möglichen Ausgestaltung eine gesteuerte Rückverlagerung des Abstützteils in die Ruhestellung erfolgen.

Die Bewegungskopplung kann bei einer Bewegung der Mundstück-Verschlusskappe in eine Öffnungsstellung zu der Spannung der Feder zwischen dem Abstützteil und der Kartusche führen, dies insbesondere gemäß einer Ausführungsform dadurch bedingt, dass zufolge des sich in die Bewegungsrichtung der Kartusche stemmenden Anschlags das Verlagerungsmaß der Kartusche in Bewegungsrichtung geringer ist als das entsprechende Verlagerungsmaß des Abstützteils. Hieraus ergibt sich eine Spannung der Feder, die mit Fortschwenken des Anschlages im Zuge des Ansaugens über das Mundstück freigegeben wird, zur weiteren Verlagerung der Kartusche (und gegebenenfalls des Zählwerks) in Bewegungsrichtung, wobei unter gehäuseseitiger Abstützung des kartuschenseitigen Ventilrohrs das kartuscheninterne Ventil entgegen der Ventilfederkraft öffnet und ein Sprühstoß ausgegeben wird.

Die in der Sprühstellung auf die Kartusche einwirkende Federkraft über die zwischen Kartusche und Gehäuse, beispielsweise Abstützteil, wirkende Feder ist hierbei größer gewählt als die durch die kartuschenseitige Ventilfeder aufzubringende Gegenkraft.

In weiterer Ausgestaltung kann das Auslöseteil zur Auslösung der Kartuschenverschiebung aus der Ausgabebereitschaftsstellung in die Ausgabestellung ohne eine Unterdruckbeaufschlagung über das Mundstück in Form eines Stößels oder eines Stiftes ausgebildet sein. Das Auslöseteil ist bevorzugt nicht Bestandteil des an den Benutzer herausgegebenen Handgerätes.

Das Auslöseteil kann zur Einwirkung auf einen im Inneren des Gehäuses angeordneten Auslöseschlitten bewegbar sein. Der Auslöseschlitten kann in dem Gehäuse schiebeverlagerbar aufgenommen sein, gegebenenfalls in eine Grundstellung unter Federbelastung vorgespannt sein. Über das durch die Öffnung eingeführte Auslöseteil kann der Auslöseschlitten aus einer Grundstellung heraus in eine Auslösestellung verlagert werden, dies gegebenenfalls unter Überwindung einer Rückstellfederkraft, in welcher Auslösestellung die Gegenkraft, bei Verharren der Kartusche in der Ausgabebereitschaftsstellung, außer Wirkung bringbar ist.

In weiterer Ausgestaltung kann, wie erwähnt, eine Ventilklappe vorgesehen sein, die durch Unterdruck bewegbar ist. Über die Ventilklappenbewegung kann beispielsweise eine mechanische Sperre der Verschiebung der Kartusche aus der Ausgabebereitschaftsstellung in die Ausgabestellung aufgehoben werden.

Im Zusammenhang mit einer Auslösung ohne eine Unterdruckbeaufschlagung über das Mundstück kann gemäß einer weiter bevorzugten Ausgestaltung vorgesehen sein, dass die Ventilklappe durch den Auslöseschlitten bewegbar ist. Entsprechend kann die Ventilklappe ausgelegt sein, zur Verschwenkung über eine Unterdruckbeaufschlagung bei üblicher Nutzung des Handgerätes und zur Verschwenkung unmittelbar über das Auslöseteil oder mittelbar über den Auslöseschlitten. Der Auslöseschlitten kann hierbei unmittelbar auf die Ventilklappe einwirken, so dass eine Verlagerung des Auslöseschlittens eine entsprechende Verlagerung der Ventilklappe zur Folge haben kann.

Die Ventilklappe kann darüber hinaus alternativ zur Aufhebung einer mechanischen Sperre auch einen Luftweg öffnen, insbesondere zum Abbau einer die Kartusche in der Ausgabebereitschaftsstellung haltenden Gegenkraft.

### Kurze Beschreibung der Zeichnungen

Nachstehend ist die Offenbarung anhand der beigefügten Zeichnung, die aber lediglich Ausführungsbeispiele darstellt, näher erläutert. Ein Teil, das nur bezogen auf eines der Ausführungsbeispiele erläutert ist und bei einem weiteren Ausführungsbeispiel aufgrund der dort herausgestellten Besonderheit nicht durch ein anderes Teil ersetzt ist, ist damit auch für dieses weitere Ausführungsbeispiel als jedenfalls mögliches vorhandenes Teil beschrieben. Die Zeichnung zeigt:
- Fig. 1: das Handgerät in perspektivischer Darstellung bei mit einer Verschlusskappe verschlossenem Mundstück;
- Fig. 2: die Vorderansicht gegen das Handgerät;
- Fig. 3: den Schnitt gemäß der Linie III-III in Figur 2 durch ein Handgerät einer ersten Ausführungsform, die Ruhestellung einer in dem Handgerät-Gehäuse aufgenommenen Kartusche betreffend;
- Fig. 4: eine schematische Seitenansicht gegen das Handgerät in der Position gemäß Figur 3;
- Fig. 5: die Herausvergrößerung des Bereiches V in Figur 4;
- Fig. 6: eine der Figur 3 im Wesentlichen entsprechende Schnittdarstellung, jedoch eine Zwischenstellung im Zuge einer Aufschwenkbewegung der Mundstück-Verschlusskappe betreffend;
- Fig. 7: eine Folgedarstellung zu Figur 6, betreffend eine Ausgabebereitschaftsstellung der Kartusche, bei einer Mundstück-Verschlusskappe in der Offenstellung;
- Fig. 8: eine der Figur 4 entsprechende Darstellung, jedoch die Situation gemäß Figur 7 betreffend;
- Fig. 9: die Herausvergrößerung des Bereiches IX in Figur 8;
- Fig. 10: eine Folgedarstellung zu Figur 7, betreffend die Situation im Moment eines mit einem Ansaugvorgang einhergehenden Sprühausstoßes einer in der Kartusche befindlichen Substanz;
- Fig. 11: das Handgerät in einer perspektivischen Explosionsdarstellung;
- Fig. 12: in einer Schnittdarstellung gemäß Figur 3 einen Ausschnitt eines Handgerätes in einer zweiten Ausführungsform, die Ruhestellung betreffend;
- Fig. 13: den Schnitt gemäß der Linie XIII-XIII in Figur 12;
- Fig. 14: eine der Figur 12 entsprechende Schnittdarstellung, betreffend die Situation gemäß Figur 10;
- Fig. 15: den Bereich XV in Figur 14, betreffend eine alternative Auslösung;

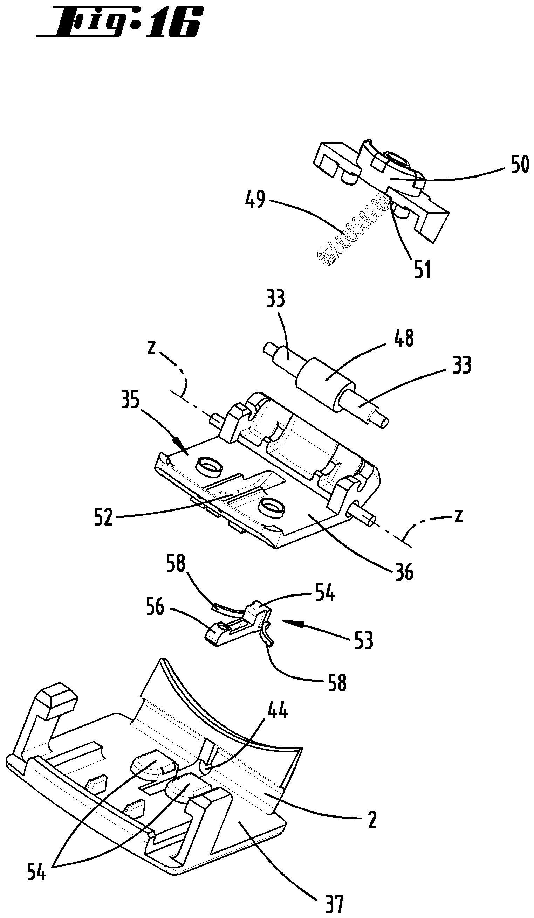

- Fig. 16: eine explosionsperspektivische Darstellung eines Teilbereiches des Handgerätes der zweiten Ausführungsform.

### Beschreibung der Ausführungsformen

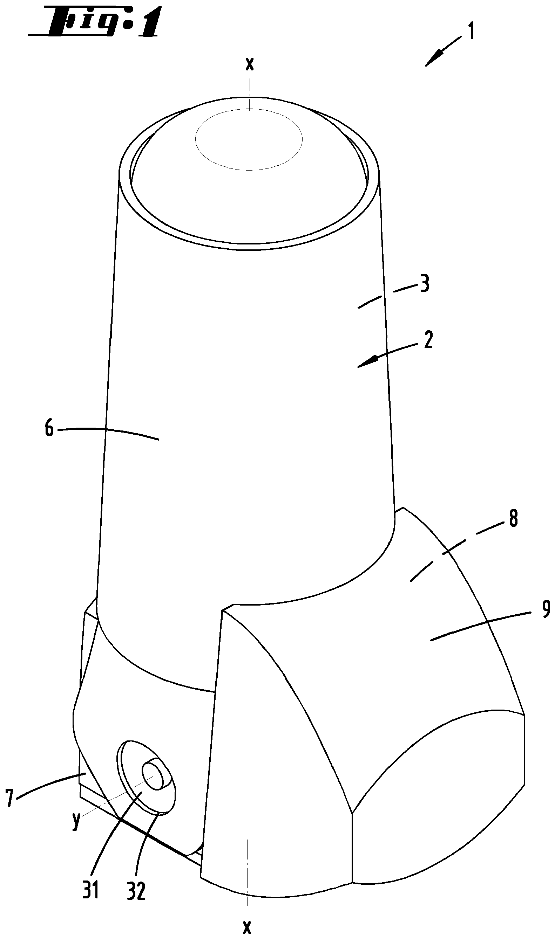

Dargestellt und beschrieben ist, zunächst mit Bezug zu Figur 1, ein Handgerät 1 zur portionierten Ausgabe sprühfähiger Substanzen, insbesondere von Inhalier-Medikamenten.

Das Handgerät 1 weist ein Gehäuse 2 auf, in welches eine die sprühfähige Substanz beinhaltende Kartusche 3 eingesetzt ist. Die Kartusche 3 ist mit Bezug auf eine in Längsrichtung der Kartusche 3 verlaufende Achse x, die im Wesentlichen auch die Längsachse des Handgerätes 1 insgesamt darstellt, in dem Gehäuse 2 axial verschiebbar.

In üblicher Weise weist der Kartuschenkopf 4 ein zentrales, sich koaxial zur Kartusche 3 erstreckendes Ventilrohr 5 auf. Über letzteres wird eine Substanzausgabe durch eine axiale Relativbewegung zwischen Kartusche 3 und Gehäuse 2 erreicht.

Das Gehäuse 2 kann, wie auch dargestellt, im Wesentlichen zweigeteilt ausgebildet sein, so weiter bestehend aus zwei in Achsrichtung übereinander angeordneten Ringteilen 6 und 7, von welchen das in den zeichnerischen Darstellungen, die im Wesentlichen auch die übliche Ausrichtung des Handgerätes 1 bei einer Anwendung entsprechen, oberen Ringteil 6 schaftartig ausgeformt ist und das untere Ringteil 7 ein etwa quer zur Schafterstreckung ausgerichtetes Mundstück 8 aufweist. Letzteres ist bei Nichtbenutzen des Handgerätes 1 durch eine Mundstück-Verschlusskappe 9 verschließbar.

Das Ventilrohr 5 der Kartusche 3 stützt sich in einem zugeordneten rohrförmigen Stützabschnitt 10 innerhalb des unteren Ringteiles 7 ab, dies bei axialer Beweglichkeit der Kartusche 3 innerhalb des die Kartusche 3 umgebenden Gehäuses 2.

Der das Ventilrohr 5 der Kartusche 3 klemmend aufnehmende, innerhalb des unteren Gehäuse-Ringteils 7 ausgeformte Stützabschnitt 10 ist mit einem gegenüber einem das Ventilrohrende aufnehmenden Abschnitt durchmesserverringerten Strömungskanal 11 versehen, welcher strömungstechnisch in Verbindung steht mit dem Ventilrohr 5, wobei das dem Ventilrohr 5 abgewandte Ende des Strömungskanals 11 in Richtung auf das Mundstück 8 weist.

Die beiden Ringteile 6 und 7 sind in den dargestellten Ausführungsbeispielen steckbar miteinander verbunden. Alternativ können die beiden Teile aber auch über ein Gewinde, beispielsweise über ein Grobgewinde mit hoher Steigung, miteinander verbunden sein.

Die Anordnung der Kartusche 3 in dem Gehäuse 2 ist weiter so gewählt, dass der Kartuschenkopf 4 in dem Gehäuse 2 etwa auf Höhe des Verbindungsbereiches zwischen dem Ringteil 6 und dem Ringteil 7 platziert ist.

Zentral unterhalb der öffnungsseitigen Stirnwand der Kartusche 3 ist in Überlappung zum Kartuschen-Ventilrohr 5 ein Zählwerk 12 beziehungsweise Schrittschaltwerk angeordnet. Dieses dient zum Registrieren und Anzeigen der durchgeführten Ausgabebetätigungen, dies in Abhängigkeit von den durchgeführten Öffnungshüben der Kartusche 3.

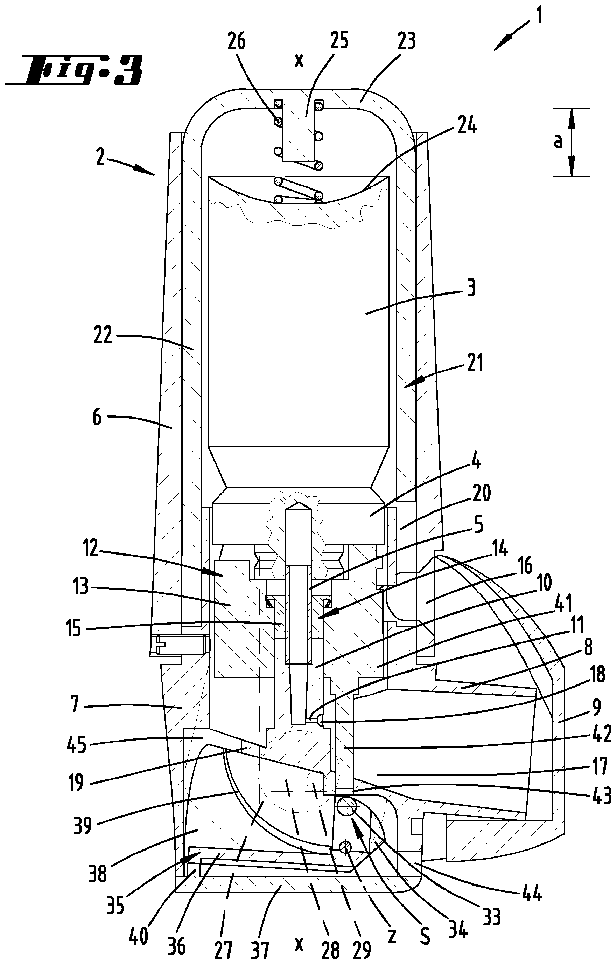

Ein solches Zählwerk ist aus der eingangs genannten WO 2009/037085 A1 bekannt. Mit Bezug beispielsweise zu der Darstellung in Figur 3 ist der Zusammenhang mit dem in der vorgenannten WO-Schrift beschriebenen Gegenstand etwa im Hinblick auf ein schematisch dargestelltes Gehäuse 13 und einem Schrittschaltfinger-Stern 14 zu erkennen. In diesem Zusammenhang ist insgesamt auch zu erkennen, dass sich der Schrittschaltfinger-Stern 14 über eine Nabe 15 auf dem Stützabschnitt 10 des unteren Ringteiles 7 abstützt, während das Zählwerk 12 insgesamt insbesondere über das Gehäuse 13 in Anlage tritt zu der zugewandten Stirnfläche des Kartuschenkopfes 4. Über ein Sichtfenster 16 ist der Skalenring des Zählwerkes 12 in einem Ausschnitt sichtbar, in welchem Ausschnitt bevorzugt die noch mindestens vorhandene Anzahl an Inhalierdosen in der Kartusche 3 angezeigt wird.

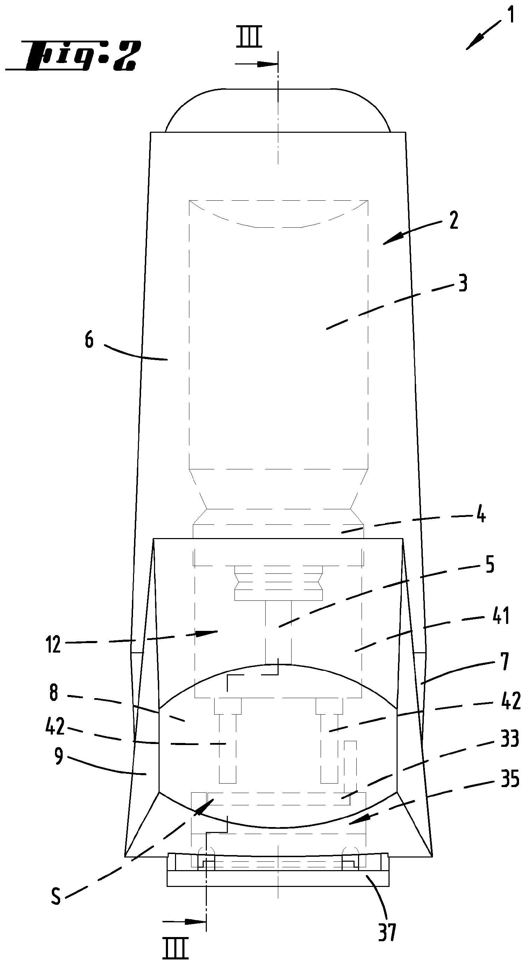

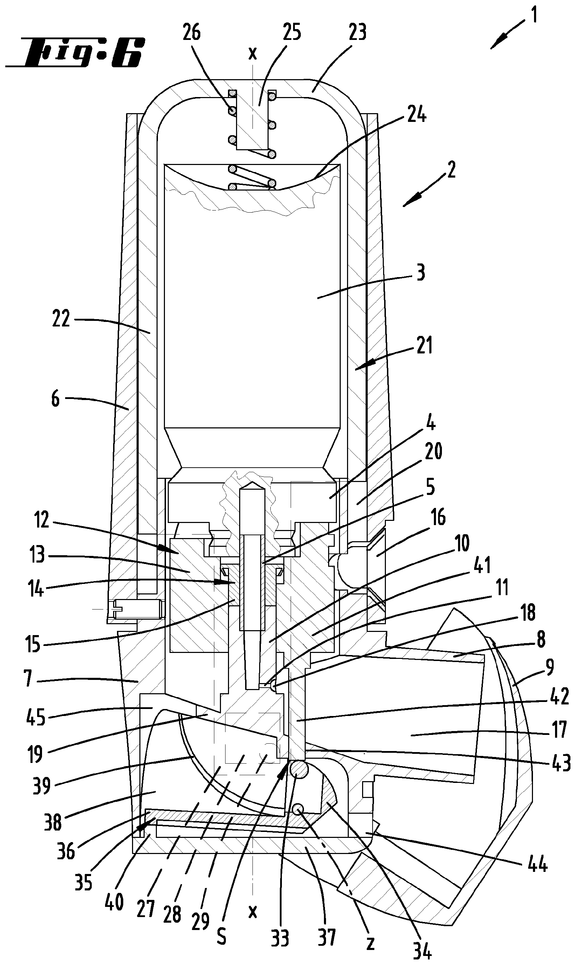

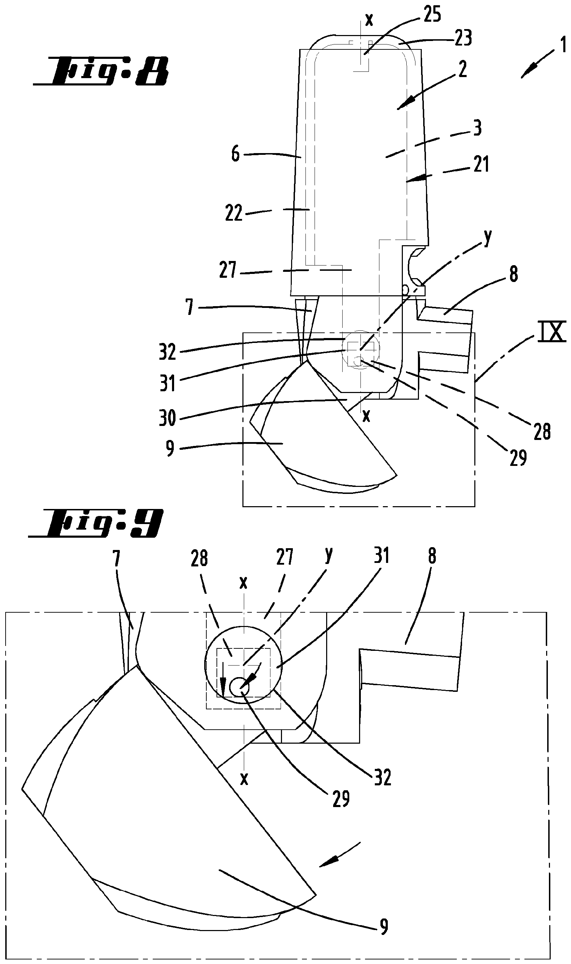

Das Sichtfenster 16 ist in dem in den Figuren 1 bis 11 dargestellten ersten Ausführungsbeispiel der, dem Mundstück 8 zugewandten Vorderseite des Handgerätes 1 zugeordnet und kann in der Nichtbenutzungsstellung beispielsweise gemäß Figur 3 von der Verschlusskappe 9 überdeckt sein.

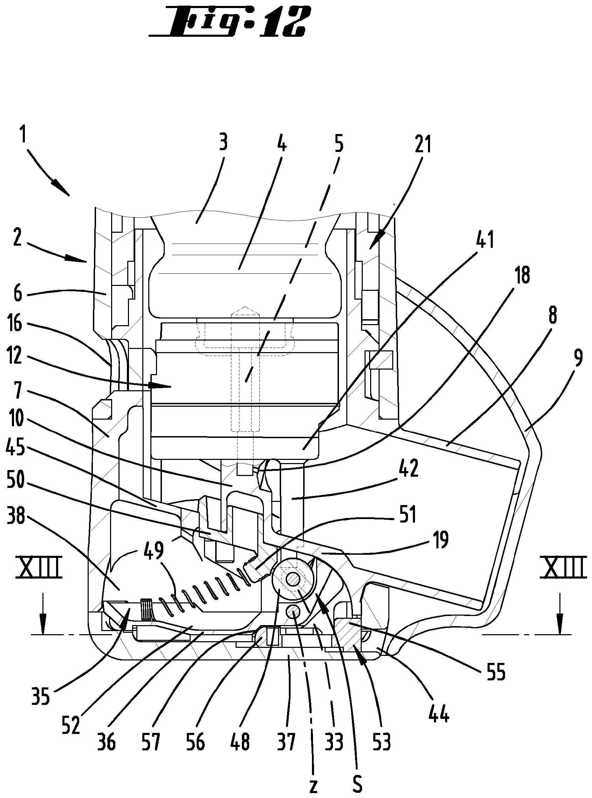

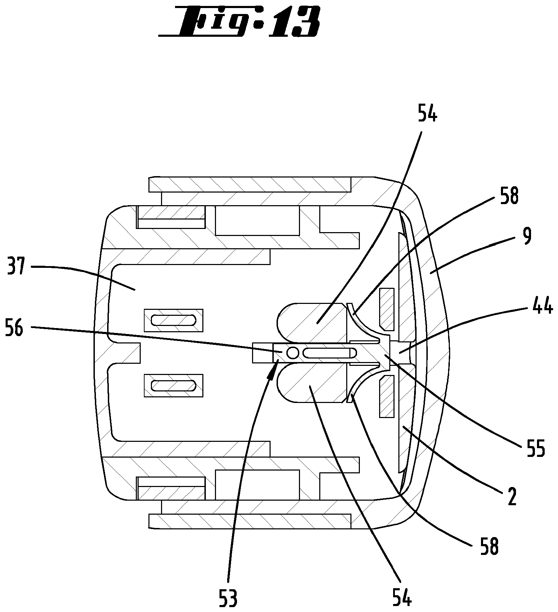

Alternativ kann das Sichtfenster 16, wie anhand des in den Figuren 12 bis 15 dargestellten zweiten Ausführungsbeispiels gezeigt, rückwärtig, d.h. auf der dem Mundstück 8 abgewandten Seite, vorgesehen sein. Der Zählwert ist hierbei unabhängig von einer Nutzungsstellung des Handgerätes 1 insgesamt, entsprechend auch unabhängig von einer Verschlusskappenstellung, ablesbar.

Der den Stützabschnitt 10 durchsetzende Strömungskanal 11 mündet in einen Luftkanal 17, der sich im Wesentlichen innerhalb des unteren Ringteiles 7, den Stützabschnitt 10 umgebend, erstreckt und in das Mundstück 8 mündet. Das in den Luftkanal 17 mündende freie Ende des Strömungskanals 11 bildet eine Sprühdüse 18.

Der Luftkanal 17 ist nach unten, entsprechend abweisend zu dem Zählwerk 12 durch einen ersten Gehäuseboden 19 begrenzt, welcher erste Gehäuseboden 19 in dem dargestellten Ausführungsbeispiel im Wesentlichen übergeht in eine mit Bezug auf die Darstellungen untere Begrenzungswandung des Mundstücks 8.

Zwischen der Außenwandung der Kartusche 3 und der Innenwandung des oberen Ringteils 6 des Gehäuses 2 ergibt sich ein Ringraum 20. In diesen greift ein topfförmig gestaltetes Abstützteil 21 mit dessen umlaufenden, konzentrisch zur Achse x verlaufenden Wandung 22. Der Topfboden des Abstützteiles 21 erstreckt sich in Art einer kuppelförmigen Decke 23 über der Kartusche 3, wobei zwischen dem Kartuschenboden 24 und der zugewandten Unterseite der Decke 23 ein in Achsrichtung betrachteter Abstand a verbleibt.

Unterseitig der Decke 23 ist mittig, die Achse x zentral aufnehmend, ein Dorn 25 angeformt. Dieser ist umfasst von einer Feder 26 in Form einer Zylinder-Druckfeder, die sich einerends an der Decke 23 des Abstützteiles 21 abstützt und anderenends auf dem zugewandten Kartuschenboden 24.

In der beispielsweise anhand des ersten Ausführungsbeispiels in Figur 3 gezeigten Ruhestellung der Kartusche 3, die der Nichtnutzungsstellung entspricht, liegt in einer bevorzugten Ausgestaltung die Feder 26 ohne Kraftbeaufschlagung auf dem Kartuschenboden 24 an. Die Feder 26 bewirkt entsprechend keine oder keine maßgebliche Belastung der Kartusche 3 in ihre Bewegungsrichtung r. Vielmehr ist die Kartusche 3 in ihrer üblichen Grundstellung gehalten, in welcher Grundstellung die Kartusche 3 gegenüber dem in dem Stützabschnitt 10 gehaltenen Ventilrohr 5 entlang der Achse x mit Bezug zu den Darstellungen nach oben in Richtung auf die Abstützteil-Decke 23 gedrängt ist, dies zufolge entsprechender anschlagbegrenzter Federrückstellung einer im Inneren der Kartusche 3 im Bereich des Ventilrohres 5 vorgesehenen Ventilfeder.

Das Abstützteil 21 ist in dem Ringraum 20 linear entlang der Achse x schiebeverlagerbar. Hierzu weist das Abstützteil 21 in dem dargestellten Ausführungsbeispiel mit Bezug auf einen Querschnitt quer zur Achse x zwei diametral gegenüberliegend angeordnete, mit der Wandung 22 bevorzugt einstückig materialeinheitlich ausgebildete Abschnitte 27 auf, die laschenartig Abschnitte der Wandung 22 über die von der Decke 23 abgewandten Randkante der Wandung 22 hinaus verlängern. Die Abschnitte 27 erstrecken sich in der in Figur 3 dargestellten Ruhestellung etwa bis in eine quer zur Achse x betrachtete Projektion zu dem ersten Gehäuseboden 19.

In dem jeweiligen Endbereich der Abschnitte 27 ist ein fensterartiger Ausschnitt oder eine fensterartige Vertiefung 28 ausgebildet, in welche ein Exzenternocken 29 der Mundstück-Verschlusskappe 9 eingreift.

Hierzu weist die Mundstück-Verschlusskappe 9 mit Blick auf die Mundstücköffnung beidseitig der Achse x jeweils einen auslegerartigen Kappenfortsatz 30, in welchem jeweils ein Exzenternocken 29 angeordnet ist. Der Exzenternocken 29 greift in die Vertiefung 28 des zugeordneten Abstützteil-Abschnitts 27 ein.

Die geometrische Drehachse y der Mundstück-Verschlusskappe 9 ergibt sich zufolge Zusammenwirkung von an den Kappenfortsätzen 30 jeweils nach außen weisenden Achsstümpfen 31, die in entsprechend positionierte und dimensionierte bohrungsartige Achsaufnahmen 32 im Gehäuse 2, insbesondere des oberen Ringteiles 6, eingreifen.

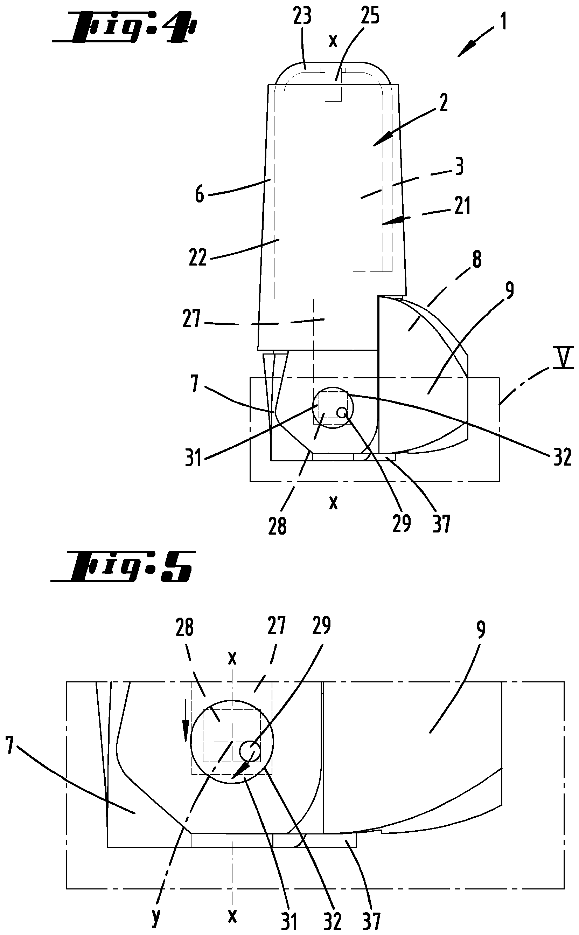

Wie beispielsweise aus der Darstellung in Figur 5 zu erkennen, ergibt sich ein exzentrischer Versatz des Exzenternockens 29 zu der Drehachse y, welche Drehachse y in dem dargestellten Ausführungsbeispiel die Achse x querend schneidet.

Mit Aufschwenken der Mundstück-Verschlusskappe 9 ist zufolge der vorbeschriebenen Ausgestaltung eine Exzenterbeaufschlagung des Abstützteiles 21 über die Kappenfortsätze 30 erreicht. Das Aufschwenken der Mundstück-Verschlusskappe 9 durch Abschwenken derselben mit Bezug zu den Darstellungen nach unten führt zu einer linearen Absenkung des Abstützteiles 21.

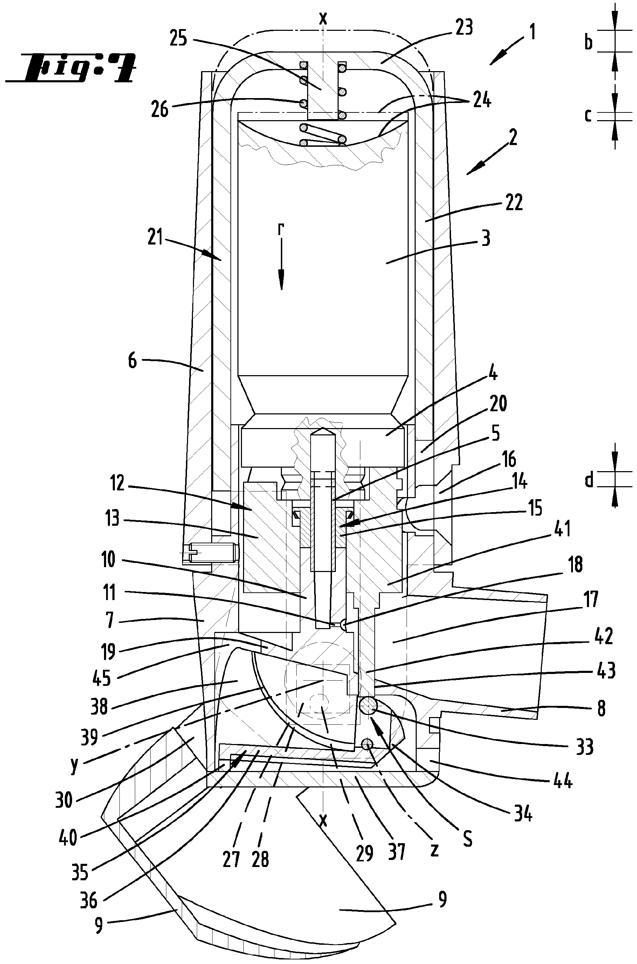

Diese lineare Absenkung des Abstützteiles 21 bewirkt über die Feder 26 eine begrenzte Linearbewegung der Kartusche 3 nach unten in Richtung auf den Stützabschnitt 10, wobei zufolge der Abstützung des Ventilrohres 5 auf dem Stützabschnitt 10 das Ventilrohr 5 entgegen der Kraft der kartuschenseitigen Feder in Richtung auf das Kartuscheninnere eintaucht. Eine Schleppmitnahme der Kartusche 3 bis in eine Stellung, in welcher das kartuscheninterne Ventil öffnet, entsprechend eine ausreichend große Relativverlagerung von Kartusche 3 und Ventilrohr 5 erreicht ist, ist zufolge einer mechanischen Sperre S zunächst unterbunden (siehe Figur 7).

Die mechanische Sperre S hindert die Kartusche 3 an einer weiteren Verlagerung und hält die Kartusche 3 in einer Ausgabebereitschaftsstellung.

Die mechanische Sperre S ist in dem dargestellten Ausführungsbeispiel durch einen Anschlag 33 gegeben. Der Anschlag 33 ist gemäß der dargestellten Ausführungsform stiftartig gestaltet mit einem kreisscheibenförmigen Querschnitt, wobei die Stablängsachse parallel verläuft zu der Drehachse y der Mundstück-Verschlusskappe 9.

Der stiftförmige Anschlag 33 ist in dem dargestellten Ausführungsbeispiel beiderends gehaltert in einem Ausleger 34 einer vorgesehenen Ventilklappe 35.

Die Ventilklappe 35 ist über eine Schwenkachse z schwenkbar in dem unteren Ringteil 7 gehaltert, welche Schwenkachse z sich in der Ruheposition der Ventilklappe 35 im Wesentlichen unterhalb mit Bezug auf die Bewegungsrichtung r der Kartusche 3 des Anschlags 33 verläuft.

Mit Bezug auf einen Querschnitt gemäß Figur 3 erstreckt sich etwa in rechtwinkliger Ausrichtung zu dem Ausleger 34 der Klappenboden 36.

Erfindungsgemäß ist die Ventilklappe 35 mit ihrem Ausleger 34 und dem Anschlag 33 in Bewegungsrichtung r der Kartusche 3 betrachtet unterhalb des ersten Gehäusebodens 19 angeordnet, insbesondere in einem zwischen dem ersten Gehäuseboden 19 und einem zweiten Gehäuseboden 37 innerhalb des unteren Ringteiles 7 begrenzten Klappenraumes 38.

Die Ventilklappe 35 kann ein Kunststoff-Spritzteil sein. Dies bietet den Vorteil der unmittelbaren Anformung eines federartig auf die Ventilklappe 35 wirkenden Rückstellarmes 39. Dieser stützt sich unterseitig an dem ersten Gehäuseboden 19 ab und drängt die Ventilklappe 35 in ihre Grundposition gemäß Figur 3, in welcher diese auf dem zweiten Gehäuseboden 37 aufliegt beziehungsweise hier an einem Stützbock 40 anliegt.

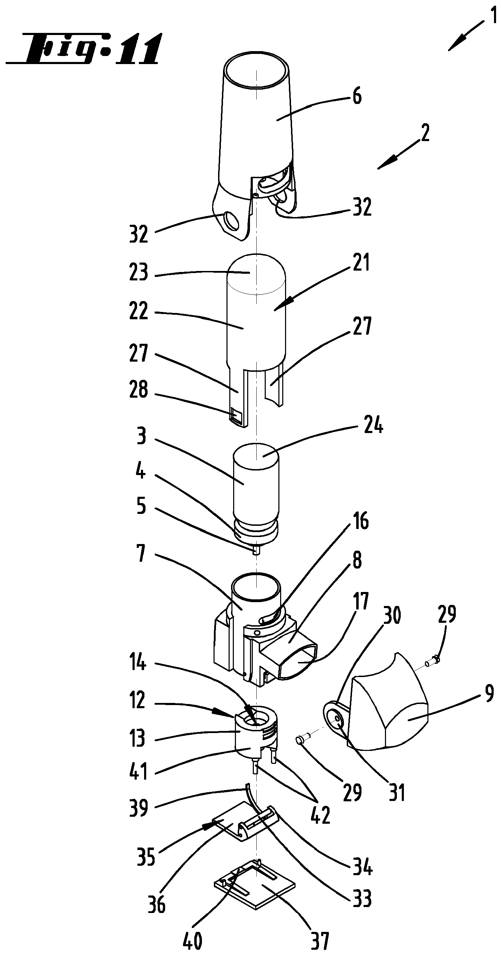

Der Anschlag 33 beziehungsweise die mechanische Sperre S wirkt insbesondere in der Ausgabebereitschaftsstellung gemäß Figur 7 zusammen mit einem Anschlagteil 41 der Kartusche 3. Dieses Anschlagteil 41 kann, wie auch dargestellt, Teil des Zählwerks 12 sein, beispielsweise des Gehäuses 13 (siehe auch Figur 11). Das Anschlagteil 41 weist zur unmittelbaren Zusammenwirkung mit dem Anschlag 33 einen Anschlagfortsatz 42 auf, der stiftartig den Luftkanal 17 in Ausrichtung der Achse x querend den ersten Gehäuseboden 19 im Bereich einer entsprechend ausgeformten und bevorzugt querschnittsangepassten Durchbrechung 43 durchsetzt.

Wie insbesondere aus der Darstellung in Figur 11 zu erkennen, sind zwei derartige Anschlagfortsätze 42 vorgesehen, die sich mit Blick auf die Mundstück-Öffnungsfläche beidseitig der Achse x erstrecken und gemeinsam mit dem Anschlag 33 zusammenwirken.

In der Ausgabebereitschaftsstellung gemäß Figur 7 stützt sich entsprechend die Kartusche 3 über das Zählwerk 12 und hierüber über die Anschlagfortsätze 42 an dem klappenseitigen Anschlag 33 ab. Die Verlagerung der Kartusche 3 entlang der Achse x ist hierdurch begrenzt, während über die Exzenterbeaufschlagung unter Nutzung der Verschlusskappe 9 der Verlagerungsweg des Abstützteiles 21 gegenüber dem Verlagerungsweg der Kartusche 3 in Bewegungsrichtung r größer ist.

In Figur 7 sind jeweils in strichpunktierter Linienart die Grundstellungen von Abstützteil 21 und Kartusche 3 dargestellt. Es ist zu erkennen, dass die lineare Verlagerungsstrecke b des Abstützteiles 21 aus der Ruhestellung heraus in die Ausgabebereitschaftsstellung einem Mehrfachen der Verlagerungsstrecke c der Kartusche 3 aus der Ruhestellung in die Ausgabebereitschaftsstellung entspricht. So kann die Verlagerungsstrecke b des Abstützteiles 21 etwa dem 1,5- bis 3-Fachen, weiter etwa dem 2- bis 2,5-Fachen der kartuschenseitigen Verlagerungsstrecke c entsprechen.

Das Differenzmaß zwischen den Verlagerungsstrecken b und c wird aufgenommen zufolge der Stauchung der Feder 26 und einer Relativverlagerung des Ventilrohres 5 zu der Kartusche 3. Zufolge der Abstützung des Ventilrohres 5 auf dem Stützabschnitt 10 und der Verlagerung der Kartusche 3 in Bewegungsrichtung r ergibt sich hier entgegen der kartuscheninternen Feder ein Eintauchen des Ventilrohres 5 über eine Verlagerungsstrecke d. Das Ventilrohr 5 ist hierbei so weit eingeführt, dass das in der Kartusche 3 vorgesehene Ventil kurz vor der Auslösung steht.

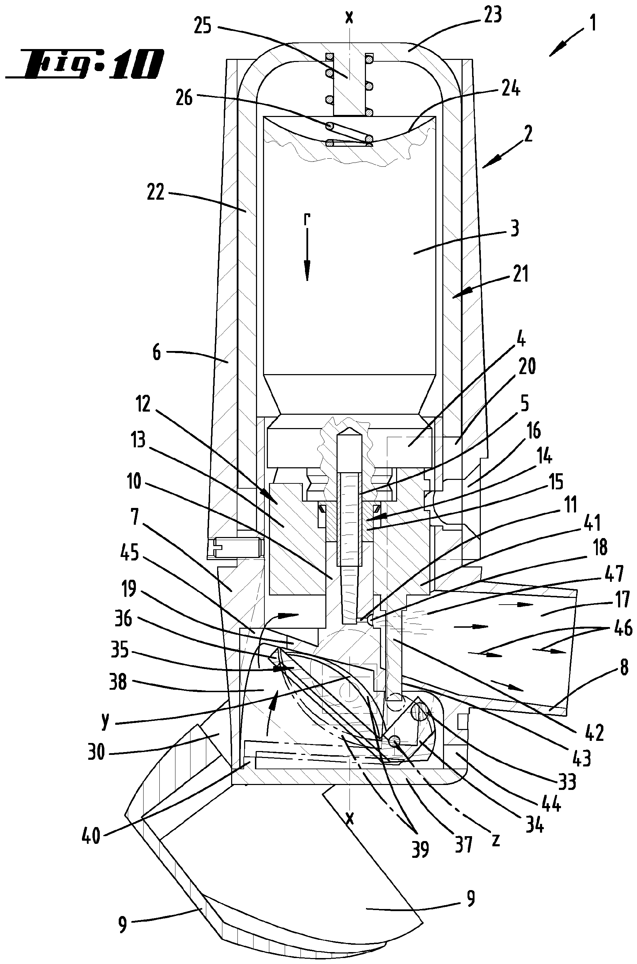

Zum Verbringen der Kartusche 3 in die Sprühstellung gemäß Figur 10 bedarf es der Aufhebung der mechanischen Sperre S. Diese Aufhebung erfolgt zufolge einer Unterdruckbeaufschlagung im Zuge eines Ansaugens von Luft durch den Benutzer. Dieser umschließt das Mundstück 8 mit seinen Lippen und atmet in üblicher Weise, wie auch bei den Handgeräten der in Rede stehenden Art, die aus dem Stand der Technik bekannt sind, tief ein. Die angesogene Luft wird hierbei über eine unterhalb des Mundstückes 8 in dem Ringteil 7 die diesbezügliche Wandung durchbrechende und in den Klappenraum 38 führende Einströmöffnung 44 angesogen und über mindestens einen Luftdurchlass 45 im ersten, entsprechend luftdurchlässigen Gehäuseboden 19 in den Luftkanal 17 geführt. Durch den sich hierbei in dem Klappenraum 38 oberseitig der Ventilklappe 35 einstellenden Unterdruck wird die Ventilklappe 35 angesogen und um die Schwenkachse z geschwenkt, dies mit Bezug zu den Darstellungen in Uhrzeigerrichtung, weiter entsprechend in Richtung auf die Unterseite des ersten Gehäusebodens 19.

Die Verschwenkung erfolgt entgegen der Rückstellkraft des Rückstellarmes 39, welcher entsprechend schwach ausgelegt ist, um allein über den Unterdruck überwunden zu werden. Allerdings ist der Rückstellarm 39 bezüglich seiner Rückstellfähigkeit so stark eingestellt, dass dieser allein eine Rückstellbewegung der Ventilklappe 35 in die Grundstellung sicherstellt.

Im Zuge der Klappenschwenkbewegung aus der Ausgabebereitschaftsstellung in die Sprühstellung schlägt der Rückstellarm 39 in der gezeigten Ausführungsform über eine Totpunktstellung hinaus über (vergleiche Darstellung in Figur 10).

Zufolge der Schwenkverlagerung der Ventilklappe 35 ergibt sich ein Abschwenken des Anschlages 33. Hierbei erweist sich eine bevorzugt um die Stabachse frei drehbare Anordnung des Anschlages 33 von Vorteil, da so ein verbessertes Abschwenken trotz Kraftbeaufschlagung über den Anschlagfortsatz 42 erfolgen kann.

Der Anschlagfortsatz 42 ist hierdurch annähernd schlagartig freigegeben. Es fehlt die entsprechende Abstützung für die Kartusche 3, die über die zufolge der zunächst unterschiedlichen Verlagerungsstrecken b und c gespannten Feder 26 in die Sprühstellung gemäß Figur 10 zwangsverlagert wird. Die Kraft der kartuscheninternen Feder wird weiter überwunden und das kartuscheninterne Ventil zufolge der entsprechenden Relativverlagerung von Kartusche 3 und Ventilrohr 5 geöffnet. Die in der Kartusche 3 bevorratete Substanz strömt portioniert durch den Strömungskanal 11 und tritt über die Sprühdüse 18 in den Luftkanal 17 und weiter in die zufolge des den Sprühausstoß auslösenden Ansaugvorganges in die Ansaugluft (dargestellt durch die Pfeile 46). Der Sprühstoß ist mit dem Bezugszeichen 47 versehen.

Mit Beendigung des Inhalationsvorganges und damit einhergehendem Abbruch des Ansaugvorganges fällt zufolge fehlender Unterdruckbeaufschlagung die Ventilklappe 35 zurück unter Verschwenkung um deren Schwenkachse z. Der Anschlag 33 kann hiernach zunächst seitlich gegen einen oder beide Anschlagfortsätze 42 treten, so dass eine endgültige Rückstellung der Ventilklappe 35 erst wieder mit Erreichen der Grundstellung, in welcher die Verschlusskappe 9 über das Mundstück 8 verschwenkt ist, erreicht werden kann.

Mit Rückschwenken der Verschlusskappe 9 zum Verschließen des Mundstückes 8 wird die Schleppmitnahme über den Exzenternocken 29 aufgehoben. Es erfolgt eine übliche Rückstellung der Kartusche 3 und über die Feder 26 des Abstützteiles 21 in die Grundstellung zufolge Entspannung der kartuschenseitigen Ventilfeder. Alternativ kann die Rückstellung insbesondere des Abstützteiles 21 auch gesteuert über den Exzenternocken 29 erfolgen.

Die Darstellungen in den Figuren 12 bis 15 zeigen eine zweite nicht erfindungsgemäße Ausführungsform eines Handgerätes 1. Bezüglich der äußeren Gestaltung und darüber hinaus auch der grundsätzlichen Funktion hinsichtlich der Handhabung des Handgerätes 1 zur Inhalation entspricht das Handgerät 1 der zweiten Ausführungsform im Wesentlichen dem der zuvor beschriebenen ersten Ausführungsform.

So ist auch hier eine Kartusche 3 in einem Abstützteil 21 aufgenommen, wobei zwischen der Kartusche 3 und der Abstützteil-Decke 23 eine Feder 26 vorgesehen ist.

Das Ventilrohr 5 ist in einem Stützabschnitt 10 des Gehäuses 2 aufgenommen. Zugeordnet dem Kartuschenkopf 4 ist auch hier ein Zählwerk 12 vorgesehen, an welchem, wie auch bei dem zuvor beschriebenen Ausführungsbeispiel, ein fingerartiger Anschlagfortsatz 42 starr angebunden ist.

Auch ist bei dieser zweiten Ausführungsform zufolge Abschwenken der Mundstück-Verschlusskappe 9 das Handgerät 1 in eine Ausgabe-Bereitschaftsstellung bringbar, in welcher sich die Kartusche 3 über das Zählwerk 12 und hierüber über den oder die Anschlagfortsätze 42 an einem Anschlag 33 abstützen.

Unterseitig des ersten Gehäusebodens 19 ist auch in dem zweiten Ausführungsbeispiel in einem Klappenraum 38 eine Ventilklappe 35 vorgesehen, welche um eine geometrische Schwenkachse z schwenkbar in dem Klappenraum 38 gelagert ist.

Der Anschlag 33 ist in dieser Ausführungsform in Art einer Walze gestaltet. Diese Walze kann, wie auch bevorzugt, um eine parallel zur Schwenkachse z der Ventilklappe 35 verlaufende geometrische Achse an der Ventilklappe 35 drehbar gehaltert sein.

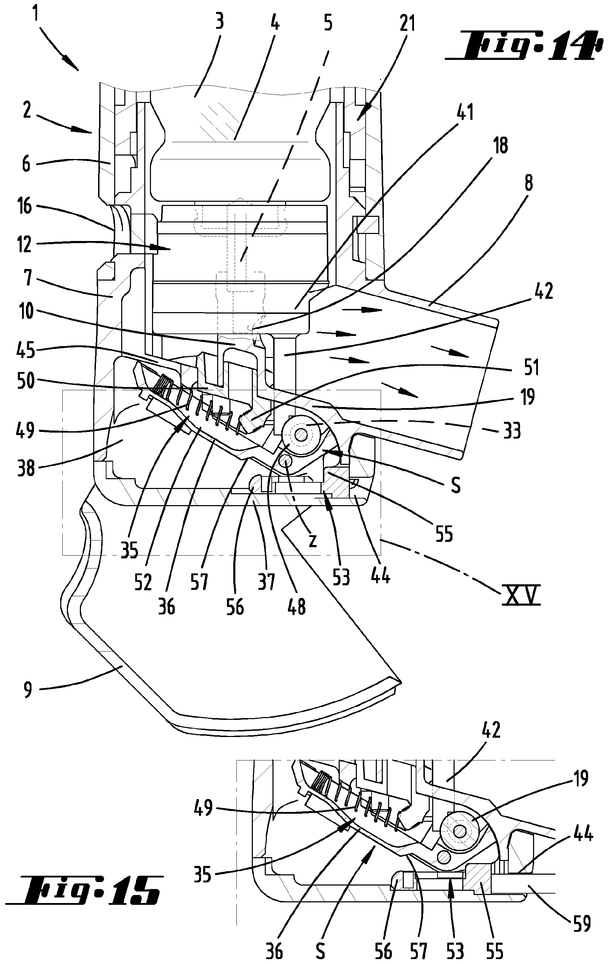

Wie weiter insbesondere aus der perspektivischen Darstellung in Figur 15 zu erkennen, kann dieser walzenartige Anschlag 33 einen in Längserstreckungsrichtung der diesbezüglichen geometrischen Drehachse mittigen durchmesservergrößerten Bereich 48 aufweisen. Dessen Durchmessermaß kann beispielsweise dem 1,5- bis 2,5-Fachen des in Längserstreckung betrachteten beidseitig des Bereiches 48 sich einstellenden Durchmessermaßes der jeweils einen Anschlag 33 bildenden Walzenbereiche entsprechen.

Anstelle eines federbaren Rückstellarmes 39, wie ein solcher in der vorbeschriebenen ersten Ausführungsform zur Anwendung kommt, kann gemäß der zweiten Ausführungsform nunmehr eine gesonderte Feder 49 vorgesehen sein. Hierbei kann es sich, wie auch dargestellt, um eine Zylinderfeder handeln, weiter beispielsweise um eine Metall-Zylinderdruckfeder.

Unterseitig des Stützabschnittes 10 kann darüber hinaus, gegebenenfalls zumindest einen Teil des ersten Gehäusebodens 19 zugleich bildend, ein Andockteil 50 vorgesehen sein. Dieses kann zumindest einen in den Klappenraum 38 einragenden Halterungszapfen 51 aufweisen, an dem ein Ende der Feder 49 gehaltert ist.

Das dem Halterungszapfen 51 abgewandte freie Ende der Feder 49 wirkt auf die Ventilklappe 35 ein derart, dass die Ventilklappe 35 in Richtung auf ihre in Figur 12 dargestellte Grundstellung belastet ist.

Dieses freie Ende der Feder 49 kann dabei, wie auch bevorzugt und dargestellt, in eine wannenartige Vertiefung 52 der Ventilklappe 35 eintauchen.

Auch bei dieser Ausführungsform ist zum Verbringen der Kartusche 3 in die Sprühstellung gemäß Figur 14 ein Aufheben der mechanischen Sperre S zwischen dem Anschlag 33 und dem Anschlagfortsatz 42 nötig. Diese Aufhebung erfolgt auch hier zufolge einer Unterdruckbeaufschlagung im Zuge eines Ansaugens von Luft durch den Benutzer über das Mundstück 8.

Die angesogene Luft wird auch hier über eine unterhalb des Mundstückes 8 in dem Ringteil 7 vorgesehene Einströmöffnung 44 angesogen und über mindestens einen Luftdurchlass 45 im ersten, entsprechend luftdurchlässigen Gehäuseboden 19 in den Luftkanal 17 geführt. Durch die sich hierbei einstellende Saugströmung, gegebenenfalls durch den sich oberseitig der Ventilklappe 35 einstellenden Unterdruck wird die Ventilklappe 35 angesogen und entgegen der Rückstellkraft der Feder 49 um die Schwenkachse z in die Stellung gemäß Figur 14 geschwenkt.

Die in dem Ausführungsbeispiel zylindrische Feder 49 kann hierbei einer in Längsrichtung der Feder 49 im Wesentlichen wirkenden Stauchung unterliegen. Darüber hinaus kann, wie auch in Figur 14 dargestellt, eine Biegung der Feder 49 insgesamt aus ihrer gestreckten Ausrichtung heraus gegeben sein.

Mit Abbruch der Saugluftströmung beziehungsweise Unterschreiten einer die Federkraft überwindenden Unterdruckbelastung wird die Ventilklappe 35 über die Feder 49 wieder zurück in ihre Ausgangsstellung gemäß Figur 12 verlagert.

Zudem kann auf dem zweiten Gehäuseboden 37 und im Wesentlichen zugeordnet unterseitig der Ventilklappe 35 ein bevorzugt schuhartiger Auslöseschlitten 53 vorgesehen sein. Dieser ist bevorzugt auf dem zweiten Gehäuseboden 37 in einer Richtung quer zur Ausrichtung der Schwenkachse z schiebeverlagerbar angeordnet, hierbei bevorzugt, in Verlagerungsrichtung betrachtet, beidseitig geführt durch auf dem Gehäuseboden 37 ausgebildete Führungsböcke 54.

An einem der Einströmöffnung 44 zugewandten Beaufschlagungsabschnitt 55 kann in Verlagerungsrichtung gegenüberliegend an dem Auslöseschlitten 53 weiter ein Auslöseabschnitt 56 ausgeformt sein. Dieser kann in einer Grundstellung gemäß Figur 12 an einer zugewandten Steuerfläche 57 im Bereich einer Unterseite der Ventilklappe 35 anliegen beziehungsweise dieser Steuerfläche 57 zugewandt angeordnet sein.

Wie weiter insbesondere aus der perspektivischen Darstellung in Figur 15 zu erkennen, kann der Auslöseschlitten 53, gegebenenfalls materialeinheitlich und einstückig angeformt, einen oder zwei Federarme 58 aufweisen, mittels welcher sich der Auslöseschlitten 53 an einem feststehenden Gehäuseteil, so beispielsweise an den Stirnflächen der Führungsböcke 54, abstützen kann. Diese Stirnflächen der Führungsböcke 54 weisen im Wesentlichen in Richtung auf das Mundstück 8, so dass sich die Federarme 58, im Wesentlichen wurzelnd im Bereich des Beaufschlagungsabschnittes 55, im Wesentlichen zwischen den Führungsböcken 54 und dem die Einströmöffnung 44 aufweisenden Wandungsabschnitt erstrecken können.

Die Federarme 58 sind dabei so ausgebildet, dass der Auslöseschlitten 53 eine federnde Belastung in Richtung auf die Grundstellung gemäß Figur 12 erfährt.

Unter Nutzung des Auslöseschlittens 53 kann insbesondere zu Test- oder Einstellungszwecken eine Auslösung eines Sprühstoßes erreicht werden, ohne dass ein Nutzer über das Mundstück 8 Luft ansaugt. Hierzu kann durch die ohnehin vorgesehene Einströmöffnung 44 ein stiftartiges Auslöseteil 59 geführt werden, über welches unter Beaufschlagung des Beaufschlagungsabschnittes 55 der Auslöseschlitten 53 entgegen der Rückstellkraft der Federarme 58 schiebeverlagert wird. Der Auslöseabschnitt 56 wirkt dabei derart mit der Steuerfläche 57 der Ventilklappe 35 zusammen, dass die Ventilklappe 35 entgegen der Rückstellkraft der Feder 49 in die den Anschlagfortsatz 42 freigebende Stellung gemäß Figur 15 erreichen kann. Auf diese Weise kann eine Verlagerung der Kartusche 3 aus der Ausgabebereitschaftsstellung in die Ausgabestellung erreicht werden und somit beispielsweise das Sprühbild geprüft und gegebenenfalls fotografiert werden. Die Erfindung betrifft auch Gestaltungsformen, bei denen einzelne der in der vorstehenden Beschreibung genannten Merkmale nicht verwirklicht sind, insbesondere soweit sie erkennbar für den jeweiligen Verwendungszweck entbehrlich sind oder durch andere technisch gleichwirkende Mittel ersetzt werden können.

**Liste der Bezugszeichen**

| | | | |
|---|---|---|---|
| 1 | Handgerät | 30 | Kappenfortsatz |
| 2 | Gehäuse | 31 | Achsstumpf |
| 3 | Kartusche | 32 | Achsaufnahme |
| 4 | Kartuschenkopf | 33 | Anschlag |
| 5 | Ventilrohr | 34 | Ausleger |
| 6, 7 | Ringteil | 35 | Ventilklappe |
| 8 | Mundstück | 36 | Klappenboden |
| 9 | Mundstück-Verschlusskappe | 37 | zweiter Gehäuseboden |
| 10 | Stützabschnitt | 38 | Klappenraum |
| 11 | Strömungskanal | 39 | Rückstellarm |
| 12 | Zählwerk | 40 | Stützbock |
| 13 | Gehäuse | 41 | Anschlagteil |
| 14 | Schrittschaltfinger-Stern | 42 | Anschlagfortsatz |
| 15 | Nabe | 43 | Durchbrechung |
| 16 | Sichtfenster | 44 | Einströmöffnung |
| 17 | Luftkanal | 45 | Luftdurchlass |
| 18 | Sprühdüse | 46 | Luft |
| 19 | erster Gehäuseboden | 47 | Sprühstoß |
| 20 | Ringraum | 48 | Bereich |
| 21 | Abstützteil | 49 | Feder |
| 22 | Wandung | 50 | Andockteil |
| 23 | Abstützteil-Decke | 51 | Halterungszapfen |
| 24 | Kartuschenboden | 52 | Vertiefung |
| 25 | Dorn | 53 | Auslöseschlitten |
| 26 | Feder | 54 | Führungsbock |
| 27 | Abschnitt | 55 | Beaufschlagungsabschnitt |
| 28 | Vertiefung | 56 | Auslöseabschnitt |
| 29 | Exzenternocken | 57 | Steuerfläche |
| 58 | Federarm | | |
| 59 | Auslöseteil | | |
| S | Sperre | | |
| a | Abstand | | |
| b | Verlagerungsstrecke | | |
| c | Verlagerungsstrecke | | |
| d | Verlagerungsstrecke | | |
| r | Bewegungsrichtung | | |
| x | Achse | | |
| y | Drehachse | | |
| z | Schwenkachse | | |

## Patentansprüche

1. Handgerät (1) zur portionierten Ausgabe sprühfähiger Substanzen, insbesondere Inhaliermedikamente, mit einem Gehäuse (2) und einer durch eine Druckbeaufschlagung relativ zu dem Gehäuse (2) in eine Ausgabestellung verschiebbaren Kartusche (3), wobei das Gehäuse (2) ein Mundstück (8) aufweist, wobei weiter die Kartusche (3) zunächst in eine Ausgabebereitschaftsstellung verschiebbar ist und bei der in Ausgabebereitschaftsstellung befindlichen Kartusche (3) durch Ansaugen ein Ausgabesprühvorgang auslösbar ist, wobei weiter die Verschiebung der Kartusche (3) in die Ausgabebereitschaftsstellung durch Spannen einer an dem Gehäuse (2) abgestützten und auf die Kartusche (3) wirkenden Feder (26) durchführbar ist, wobei die auf die Kartusche (3) einwirkende Feder (26) in die Lage versetzt wird, einen Sprühvorgang durchzuführen, hieran aber durch eine Gegenkraft gehindert ist, welche Gegenkraft durch den Benutzer zufolge einer Unterdruckbeaufschlagung durch das Mundstück (8) des Handgerätes (1) außer Wirkung bringbar ist, wobei durch den Unterdruck eine Ventilklappe (35) bewegt wird, die eine mechanische Sperre (S) der Verschiebung der Kartusche (3) aus der Ausgabebereitschaftsstellung in die Ausgabestellung aufhebt, **dadurch gekennzeichnet, dass** die Ventilklappe (35) in der Bewegungsrichtung (r) der Kartusche (3) aus der Ruhestellung in die Sprühstellung unterhalb eines ersten luftdurchlässigen Gehäusebodens (19) des Gehäuses (2) angeordnet ist und dass der Gehäuseboden (19) einen die Ventilklappe (35) aufnehmenden Raum zu dem Mundstück (8) trennt.

2. Handgerät nach Anspruch 1, **dadurch gekennzeichnet, dass** die Ventilklappe (35) in einer Bewegungsrichtung (r) der Kartusche (3) aus der Ruhestellung in die Sprühstellung unterhalb der Kartusche (3) angeordnet ist.

3. Handgerät nach einem der vorhergehenden Ansprüche, **dadurch gekennzeichnet, dass** das Gehäuse (2) eine Sprühdüse (18) aufweist, aus der in der Sprühstellung der Kartusche (3) die Substanz austritt.

4. Handgerät nach Anspruch 3, **dadurch gekennzeichnet, dass** die Sprühdüse (18) in einen teilweise durch das Mundstück (8) gebildeten Luftkanal (17) liefert.

5. Handgerät nach einem der Ansprüche 3 oder 4, **dadurch gekennzeichnet, dass** der erste Gehäuseboden (19) unterhalb der Sprühdüse (18) ausgebildet ist.

6. Handgerät nach Anspruch 4, **dadurch gekennzeichnet, dass** der erste Gehäuseboden (19) als Teil einer unteren Wandung des Luftkanals (17) gebildet ist.

7. Handgerät nach einem der vorhergehenden Ansprüche, **dadurch gekennzeichnet, dass** einer oder jeder der Luftdurchlässe (45) im ersten Gehäuseboden (19) kleiner ausgebildet ist als die Ventilklappe (35).

8. Handgerät nach einem der vorhergehenden Ansprüche, **dadurch gekennzeichnet, dass** die mechanische Sperre (S) der Bewegung der Kartusche (3) durch einen Anschlag (33) gegeben ist.

9. Handgerät nach Anspruch 8, **dadurch gekennzeichnet, dass** der Anschlag (33) mit der Ventilklappe (35) verschwenkbar ist, zur Freigabe der Kartuschenbewegung.

10. Handgerät nach einem der Ansprüche 8 oder 9, **dadurch gekennzeichnet, dass** die Kartusche (3) ein Anschlagteil (41) beaufschlagt, wobei, bevorzugt, die Ventilklappe (35) den Anschlag (33) aufweist und dass das Anschlagteil (41) zur Zusammenwirkung mit der Ventilklappe (35) ausgebildet ist.

11. Handgerät nach Anspruch 10, **dadurch gekennzeichnet, dass** das Anschlagteil (41) einen den Luftkanal (17) des Handgerätes (1) durchsetzenden Anschlagfortsatz (42) aufweist.

12. Handgerät nach einem der Ansprüche 10 oder 11, **dadurch gekennzeichnet, dass** das Anschlagteil (41) Teil eines durch die Kartusche (3) bei der Bewegung in die Sprühstellung bewegten Zählwerks (12) ist.

13. Handgerät nach einem der vorhergehenden Ansprüche, **dadurch gekennzeichnet, dass** das Gehäuse (2) zur Spannung der Feder (26) ein relativ zu der Kartusche (3) beaufschlagbares Abstützteil (21) für die Feder (26) aufweist, wobei, bevorzugt, das Handgerät (1) eine Mundstück-Verschlusskappe (9) aufweist und dass die Mundstück-Verschlusskappe (9) mit dem Abstützteil (21) bewegungsgekoppelt ist.

14. Handgerät nach Anspruch 13, **dadurch gekennzeichnet, dass** die Bewegungskopplung durch eine Exzenterbeaufschlagung eines Kappenfortsatzes (30) gegeben ist.

15. Handgerät nach einem der Ansprüche 13 oder 14, **dadurch gekennzeichnet, dass** die Bewegungskopplung bei einer Bewegung der Mundstück-Verschlusskappe (9) in eine Öffnungsstellung zu einer Spannung der Feder (26) führt.

## Claims

1. Hand-held device (1) for the portioned dispensing of sprayable substances, in particular inhalation medicaments, having a housing (2) and a cartridge (3) which can be displaced into a dispensing position relative to the housing (2) by the application of pressure, the housing (2) having a mouthpiece (8), wherein further the cartridge (3) is initially displaceable into a dispensing readiness position and, with the cartridge (3) in the dispensing readiness position, a dispensing spraying process can be triggered by suction, wherein further the displacement of the cartridge (3) into the dispensing readiness position can be carried out by tensioning a spring (26) supported on the housing (2) and acting on the cartridge (3), wherein the spring (26) acting on the cartridge (3) is enabled to but is prevented from doing so by a counterforce, which counterforce can be rendered inoperative by the user as a result of negative pressure being applied by the mouthpiece (8) of the hand-held device (1), the negative pressure moving a valve flap (35) which cancels a mechanical block (S) on the displacement of the cartridge (3) from the dispensing-readiness position into the dispensing position, **characterized in that** the valve flap (35) is arranged in the direction of movement (r) of the cartridge (3) from the rest position into the spray position below a first air-permeable housing base (19) of the housing (2) and that the housing base (19) separates a space accommodating the valve flap (35) from the mouthpiece (8).

2. Hand-held device according to claim 1, **characterized in that** the valve flap (35) is arranged below the cartridge (3) in a direction of movement (r) of the cartridge (3) from the rest position into the spraying position.

3. Hand-held device according to one of the preceding claims, **characterized in that** the housing (2) has a spray nozzle (18) from which the substance emerges in the spray position of the cartridge (3).

4. A hand-held device according to claim 3, **characterized in that** the spray nozzle (18) delivers into an air channel (17) partially formed by the mouthpiece (8).

5. Hand-held device according to one of claims 3 or 4, **characterized in that** the first housing base (19) is formed below the spray nozzle (18).

6. A hand-held device according to claim 4, **characterized in that** the first housing bottom (19) is formed as part of a lower wall of the air duct (17).

7. A hand-held device according to any one of the preceding claims, **characterized in that** one or each of the air passages (45) in the first housing base (19) is formed smaller than the valve flap (35).

8. Hand tool according to one of the preceding claims, **characterized in that** the mechanical block (S) of the movement of the cartridge (3) is given by a stop (33).

9. Hand tool according to claim 8, **characterized in that** the stop (33) is pivotable with the valve flap (35) to release the cartridge movement.

10. Hand-held device according to one of claims 8 or 9, **characterized in that** the cartridge (3) acts on a stop part (41), wherein, preferably, the valve flap (35) comprises the stop (33), and **in that** the stop part (41) is designed to cooperate with the valve flap (35).

11. Hand-held device according to claim 10, **characterized in that** the stop part (41) has a stop extension (42) passing through the air duct (17) of the hand-held device (1).

12. A hand-held device according to any one of claims 10 or 11, **characterized in that** the stop member (41) is part of a counter (12) moved by the cartridge (3) when moving to the spray position.

13. Hand-held device according to one of the preceding claims, **characterized in that** the housing (2) has a support part (21) for the spring (26) which can be acted upon relative to the cartridge (3) in order to tension the spring (26), wherein, preferably, the hand-held device (1) has a mouthpiece closure cap (9) and **in that** the mouthpiece closure cap (9) is motion-coupled to the support part (21).

14. Hand tool according to claim 13, **characterized in that** the movement coupling is provided by an eccentric impact of a cap extension (30).

15. A handset according to any one of claims 13 or 14, **characterized in that** the motion coupling results in tensioning of the spring (26) upon movement of the mouthpiece closure cap (9) into an open position.

## Revendications

1. Appareil manuel (1) pour la distribution en portions de substances pulvérisables, en particulier de médicaments à inhaler, avec un boîtier (2) et une cartouche (3) pouvant être déplacée par rapport au boîtier (2) dans une position de distribution par une sollicitation en pression, le boîtier (2) présentant un embout (8), la cartouche (3) pouvant tout d'abord être déplacée dans une position de préparation à la distribution et une opération de pulvérisation de distribution pouvant être déclenchée par aspiration lorsque la cartouche (3) se trouve dans la position de préparation à la distribution, le déplacement de la cartouche (3) dans la position de préparation à la distribution pouvant en outre être effectué en tendant un ressort (26) appuyé sur le boîtier (2) et agissant sur la cartouche (3), le ressort (26) agissant sur la cartouche (3) étant mis en position, d'effectuer un processus de pulvérisation, mais en est empêché par une force antagoniste, laquelle force antagoniste peut être rendue inopérante par l'utilisateur suite à une sollicitation par dépression à travers l'embout (8) de l'appareil manuel (1), la dépression déplaçant un clapet de soupape (35) qui supprime un blocage mécanique (S) du déplacement de la cartouche (3) de la position d'attente de distribution dans la position de distribution, **caractérisé en ce que** le clapet de soupape (35) est disposé dans la direction de déplacement (r) de la cartouche (3) de la position de repos à la position de pulvérisation en dessous d'un premier fond de boîtier (19) perméable à l'air du boîtier (2) et **en ce que** le fond de boîtier (19) sépare un espace recevant le clapet de soupape (35) de l'embouchure (8).

2. Appareil manuel selon la revendication 1, **caractérisé en ce que** le clapet (35) est disposé en dessous de la cartouche (3) dans une direction de déplacement (r) de la cartouche (3) de la position de repos à la position de pulvérisation.

3. Appareil portatif selon l'une des revendications précédentes, **caractérisé en ce que** le boîtier (2) comporte une buse de pulvérisation (18) par laquelle la substance sort lorsque la cartouche (3) est en position de pulvérisation.

4. Appareil à main selon la revendication 3, **caractérisé en ce que** la buse de pulvérisation (18) délivre dans un canal d'air (17) formé en partie par l'embout buccal (8).

5. Appareil portatif selon l'une des revendications 3 ou 4, **caractérisé en ce que** le premier fond de boîtier (19) est formé en dessous de la buse de pulvérisation (18).

6. Appareil portatif selon la revendication 4, **caractérisé en ce que** le premier fond de boîtier (19) est formé comme partie d'une paroi inférieure du canal d'air (17).

7. Appareil portatif selon l'une des revendications précédentes, **caractérisé en ce que** l'un ou chacun des passages d'air (45) dans le premier fond de boîtier (19) est plus petit que le clapet de soupape (35).

8. Appareil manuel selon l'une des revendications précédentes, **caractérisé en ce que** le blocage mécanique (S) du mouvement de la cartouche (3) est donné par une butée (33).

9. Appareil manuel selon la revendication 8, **caractérisé en ce que** la butée (33) peut pivoter avec le clapet de soupape (35), pour libérer le mouvement de la cartouche.

10. Appareil manuel selon l'une des revendications 8 ou 9, **caractérisé en ce que** la cartouche (3) sollicite une pièce de butée (41), dans lequel, de préférence, le clapet (35) comprend la butée (33) et **en ce que** la pièce de butée (41) est adaptée pour coopérer avec le clapet (35).

11. Appareil à main selon la revendication 10, **caractérisé en ce que** la pièce de butée (41) présente un prolongement de butée (42) traversant le canal d'air (17) de l'appareil à main (1).

12. Appareil manuel selon l'une des revendications 10 ou 11, **caractérisé en ce que** la pièce de butée (41) fait partie d'un compteur (12) déplacé par la cartouche (3) lors de son déplacement vers la position de pulvérisation.

13. Appareil à main selon l'une des revendications précédentes, **caractérisé en ce que** le boîtier (2) présente, pour la tension du ressort (26), une pièce d'appui (21) pour le ressort (26) pouvant être sollicitée par rapport à la cartouche (3), l'appareil à main (1) présentant, de préférence, un capuchon de fermeture d'embout (9) et **en ce que** le capuchon de fermeture d'embout (9) est couplé en mouvement avec la pièce d'appui (21).

14. Appareil manuel selon la revendication 13, **caractérisé en ce que** le couplage de mouvement est donné par une sollicitation par excentrique d'un prolongement de capuchon (30).

15. Appareil manuel selon l'une des revendications 13 ou 14, **caractérisé en ce que** le couplage de mouvement entraîne une tension du ressort (26) lors d'un mouvement du capuchon de fermeture de l'embout (9) vers une position d'ouverture.
